# EUROPEAN PATENT APPLICATION

(11) **EP 1 308 172 A1**
(43) Date of publication of application: **07.05.2003**
(21) Application number: 02023121.3
(22) Date of filing: 15.10.2002
(51) Int. Cl.: A61L 2/00, A61L 2/10, A61M 1/36

(54) **Method for protein-preserving purification of contaminated biological liquids**

(30) Priority: 25.10.2001 DE 10152159
(71) Applicant: Aventis Behring GmbH, 35002 Marburg (DE)
(72) Inventor: Lengsfeld, Thomas, 35041 Marburg (DE); Braun, Andre, 76889 Babelroth (DE); Oliveros-Braun, Esther, 76889 Babelroth (DE)

(57) **Abstract**

A method for protein-preserving purification and/or sterilization of contaminated biological liquids is described, in which the biological liquid is irradiated with UV radiation of 260 to 300 nm wavelength for a sufficient period of time.

## Description

The present invention relates to a method for protein-preserving purification and/or sterilization of contaminated biological liquids by the action of UV radiation. The invention relates in particular to the in-vitro purification and/or sterilization of products obtained from blood, such as, for example, serum, plasma, blood clotting factors, or else to the in-vitro purification and/or sterilization of other therapeutics such as vaccines or of pharmaceuticals obtained from cell cultures.

The purification and sterilization of biological liquids is a task which becomes more and more important in the modern production of pharmaceuticals, since said liquids may be contaminated by viruses (such as, for example, HIV), bacteria, fungi, yeasts, prions and other microorganisms, which may be found in therapeutic protein solutions, even in the case of pharmaceuticals produced by genetic engineering, and which may be the cause of life-threatening diseases. Some of these contaminations are only detectable with great difficulty, and in some cases there are not even useful detection methods.

Therefore, numerous studies have already been carried out with the aim of eliminating the known and unknown pathogens in sera or other biological liquids.

In this connection, it is crucial to find methods which destroy or inactivate the pathogens without damaging, at the same time, therapeutically valuable proteins. The international patent application WO 97/33629 has disclosed a method for purifying and sterilizing contaminated biological liquids and sera, in which these are subjected to UV radiation of a wavelength of 250 nm or shorter for a sufficient period of time. The selection of this UV wavelength was based on the finding that DNA- and RNA-containing solutions have a peak absorption between 240 and 260 nm.

Therefore it is possible to damage the DNA or RNA of pathogenic microorganisms at this wavelength. In contrast, proteins and protein-containing solutions have a peak absorption between 270 and 290 nm so that they should be less damaged by UV radiation of a wavelength of 250 nm (+/- 10 nm).

UV radiation is generally divided into four different regions, i.e. UVA, UVB, UVC and vacuum UV radiation, vacuum UV radiation having the shortest wavelength. UVC radiation of a wavelength of about 254 nm, which is suitable for inactivating micro-organisms, is emitted, for example, by a low-pressure mercury lamp. UV radiation of this wavelength destroys nucleic acid bonds and thus inactivates the genetic material of micro-organisms.

However, UV radiation of this wavelength region (UVC) may also destroy many valuable proteins, amino acids, enzymes and other important components of biological liquids. In order to avoid these difficulties, it is therefore suggested in the international application WO 97/33629 that the UV radiation acting on the biological liquid is limited to a narrow spectral range by selecting suitable filters and photometric grids and that no UV wavelengths are used which are absorbed by the therapeutically valuable proteins.

Nevertheless, the method described there cannot completely prevent damaging of said proteins, since radiation is absorbed by all components of a given sample which contain a suitable chromophore for a given wavelength or range of wavelengths of excitation, in accord with Lambert-Beer's law. In addition, such conventional radiation sources may emit infrared radiation which may heat up the biological liquid when applied for a relatively long period. In addition, particular UVC lamps may produce ozone which can destroy oxidation-sensitive proteins.

In another, more recent application (US 5,981,163) such damage was prevented by addition of radical traps and quenchers of reactive oxygen species. In fact, the application focuses on photoinitiated and photosensitized processes aimed at the oxidative degradation of such viruses bacteria, fungi, yeasts, prions and other micro-organisms. The inventors claimed protection for their invention concerning photoinitiated sterilization processes under normal atmosphere, hence using dissolved molecular oxygen as a substrate to produce reactive intermediates, such as singlet oxygen or superoxide anion, but also peroxyl radicals as secondary products of the additon of molecular oxygen to C-centered radicals produced by photolytic action.

A method for protein-preserving purification and/or sterilization of contaminated biological liquids has been found, in which the biological liquid is exposed to UV radiation of a wavelength of from 260 to 300 nm, preferably a wavelength of 282 +/- 15 nm. Contrary to the previous view that UV radiation of wavelengths located within the peak absorption of most proteins should have a particularly high damaging potential, it was surprisingly found that UV radiaton in the range of 282 +/- 15 nm causes only negligible damage to the pro teins but instead complete inactivation of the pathogenic micro-organisms.

UV radiation of a wavelength of 282 +/- 15 nm, which is required for the method of the invention, can be filtered from the emission of a xenon, deuterium or mercury radiation source or generated using an appropriate (e.g. excimer) laser. A non-coherent XeBr excimer radiation source emitting at 282 +/- 15 nm with low photonic irrradiance (e.g. [einstein s⁻¹ cm⁻²]) is particularly suitable.

In taking into account the high absorbance of such liquids in the wavelength range of excitation, the method is carried out in a suitable way to expose a relatively high fraction of the total sample volume to UV radiation of wavelength 282 +/- 15 nm preferably under an non inert or inert, preferably protective, gas in an appropriate container. The biological liquid can be irradiated batchwise or, preferably, continuously by conducting the biological liquid to be decontaminated past one of the above mentioned UV radiation sources, it also being possible for the radiation source to be in the liquid (e.g. immersion type reactor). The duration of UV irradiation depends on the extent of contamination, photonic irradiance and reactor design. Other important parameters are the optical path length in the biological liquid, the potential of said liquid for absorbing UV radiation and the irradiation time. Irradiation time (or residence time in the photochemical reactor) may last from several seconds to minutes.

The method of the invention has proved to be very suitable for decontaminating biological liquids and has been particularly successful in inactivating coated and uncoated viruses. In particular, it was possible to completely inactivate parvoviruses, reoviruses, HI vi ruses, EMC viruses and hepatitis A and hepatitis B viruses.

A combination of the treatment of contaminated biological liquids with other known virucidal methods, for example the solvent/detergent method, can increase the safety of biological liquids, vaccines and therapeutics produced from blood still further.

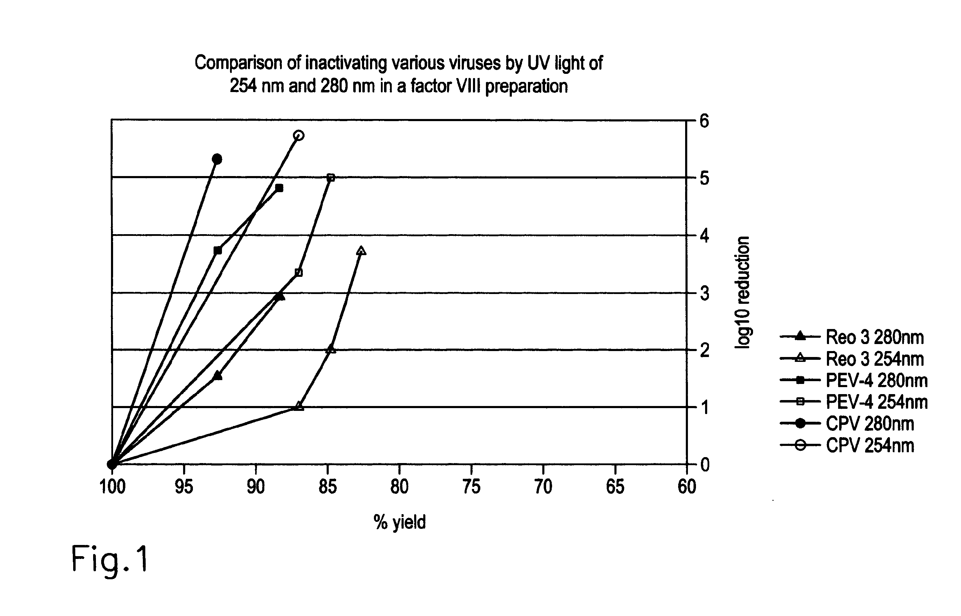

Fig. 1 shows a comparison of inactivating reoviruses, PEV (porcine enteritis virus) and CPV (canine parvovirus) viruses both by UV radiation of a wavelength of 254 nm and by UV radiation of a wavelength of 282 nm in a factor VIII preparation. This indicates that a considerably higher degree of inactivation of said viruses can be achieved at a wavelength of 282 +/- 15 nm under otherwise identical conditions.

## Claims

1. A method for protein-preserving purification and/or sterilization of contaminated biological liquids, which comprises irradiating the biological liquid with UV radiation of 260 to 300 nm wavelength for a sufficient period of time.

2. The method as claimed in claim 1, wherein the biological liquid used is plasma, serum, blood, vaccines or protein solutions.

3. The method as claimed in claims 1 and 2, wherein the UV radiation has a wavelength of 282 +/- 15 nm.

4. The method as claimed in claims 1 to 3, wherein the ultraviolet radiation is emitted from an excimer, deuterium, ion or mercury or doped (i.e. added metal iodides) radiation source.

5. The method as claimed in claims 1 to 4, wherein an excimer radiation source with a UV radiation of wavelength 280 +/- 15 nm is used.

6. The method as claimed in claims 1 to 5, wherein irradiation is made under the exclusion of molecular oxygen.

7. The method as claimed in claims 1 to 5, wherein irradiation is made in presence of molecular oxygen.

8. The method as claimed in claims 1 to 6, wherein irradiation is under a blanket of inert gas, such as nitrogen, argon, helium or carbon dioxide.

9. The method as claimed in claims 1 to 8, wherein the biological liquid is irradiated batchwise with the UV radiation.

10. The method as claimed in claims 1 to 8, wherein the biological liquid is irradiated continuously with the UV radiation, while passing through a hollow body permeable to UV radiation.
